# EUROPÄISCHE PATENTANMELDUNG

(11) **EP 2 548 502 A1**
(43) Veröffentlichungstag der Anmeldung: **23.01.2013**
(21) Anmeldenummer: 12169500.1
(22) Anmeldetag: 25.05.2012
(51) Int. Cl.: A61B 5/024, A61B 5/0408

(54) **Brustgürtel**

(30) Priorität: 20.07.2011 DE 102011108145
(71) Anmelder: Müller & Sebastiani Elektronik GmbH, 85521 Ottobrunn (DE)
(72) Erfinder: Neumüller, Werner, 85570 Unterschwillach (DE)
(74) Vertreter: Manitz, Finsterwald & Partner GbR

(57) **Zusammenfassung**

Die Erfindung betrifft einen Brustgürtel zur Aufnahme von EKG-Signalen mit einer Innen- und einer Außenseite sowie zumindest zwei innenseitig angeordneten, elektrisch leitenden, flexiblen Elektroden, welche ein Polymer enthalten und über Leitungen mit je einem Kontaktabschnitt verbunden sind. Der Brustgürtel besitzt einen Gürtelabschnitt, der in Sandwichbauweise aufgebaut ist und zwei Isolierschichten aufweist, die zwischen zwei Textilschichten eingebettet sind. Der Gürtelabschnitt weist ferner zwei in Gürtellängsrichtung voneinander beabstandete Elektroden auf, wobei jeder Elektrode eine zwischen den beiden Isolierschichten eingebettete, zum jeweiligen Kontaktabschnitt führende elektrische Leitung zugeordnet ist. Die innere Isolierschicht sowie die innenseitige Textilschicht besitzen miteinander fluchtende Durchbrechungen, durch welche sich die Elektroden hindurch erstrecken.

## Beschreibung

Die Erfindung betrifft einen Brustgürtel zur Aufnahme von EKG-Signalen mit einer Innen- und einer Außenseite sowie zumindest zwei innenseitig angeordneten, elektrisch leitenden, flexiblen Elektroden, welche ein Polymer enthalten und über Leitungen mit je einem Kontaktabschnitt verbunden sind.

Derartige Brustgürtel sind beispielsweise aus der DE 10 2009 052 615 A1 der Anmelderin bekannt und dienen als Messaufnehmer im ambulanten sowie im klinischen Bereich in Verbindung mit entsprechenden EKG-Geräten der Aufnahme von Langzeit- oder Belastungs-EKG-Daten. Weiterhin werden derartige Messaufnehmer für EKG-Daten auch im Sportbereich zur Überwachung der Herzfrequenz eines Sportlers und im Bereich der Telemedizin genutzt, bei der ein Patient selbständig eine Untersuchung am eigenen Körper vornimmt und Messdaten zur Diagnose durch eine Sendeeinheit an eine räumlich entfernte Diagnosestation übertragen werden.

Üblicherweise werden zumindest zwei Elektroden im Brustbereich und mindestens eine Elektrode im Rückenbereich des Brustgürtels angeordnet, um so aussagekräftige und voneinander in relevantem Maße abweichende Potentialunterschiede ermitteln zu können. Alternativ kann der Brustgürtel aber auch nur zwei oder mehr als drei Elektroden aufweisen. Der Brustgürtel kann in jedem Fall zwei freie Enden besitzen, die jeweils einen Kontaktabschnitt bilden und im Bereich dieser Kontaktabschnitte elektrisch mit einer Sendeeinheit koppelbar sind. Durch das Verbinden der beiden Kontaktabschnitte mit der Sendeeinheit werden gleichzeitig die beiden freien Enden des Brustgürtels mechanisch miteinander verbunden, so dass die Sendeeinheit zusätzlich eine Verschlussfunktion für den Brustgürtel übernimmt.

Wie bereits erwähnt, sind zwischen den flexiblen Elektroden und dem ihr jeweils zugeordneten Kontaktabschnitt elektrische Leitungen vorgesehen. Diese Leitungen können gemäß Stand der Technik in den Brustgürtel integriert werden, wobei es sich hinsichtlich des Tragekomforts des Brustgürtels positiv auswirkt, wenn diese Leitungen zumindest teilweise aus textilem Material bestehen. Nachteilig an derartigen Leitungen ist jedoch die Tatsache, dass ihre Leitfähigkeit durch Schweiß oder wiederholtes Waschen des Brustgürtels beeinträchtigt werden kann, so dass dann keine zuverlässige Signalübertragung zwischen den Elektroden und den Kontaktabschnitten mehr gewährleistet ist.

Wenn unterschiedlich weit von nahe beieinander liegenden Kontaktabschnitten beabstandete Elektroden über Leitungen mit diesen Kontaktabschnitten verbunden werden, verläuft zwangsläufig die Leitung der weiter beabstandeten Elektrode entlang der anderen, weniger weit von den Kontaktabschnitten beabstandeten Elektrode. Wenn sich zwischen dieser Elektrode und der Leitung als elektrischer Isolator lediglich nicht leitfähiges textiles Material befindet, besteht die Gefahr, dass sich dieses Material mit Schweiß vollsaugt, so dass zwischen der Elektrode und der Leitung Spannungsbrücken entstehen, die zu einer Verfälschung der ermittelten Messdaten führen. Um dies zu vermeiden, ist es aus dem Stand der Technik bekannt, die Leitung zumindest im Bereich der Elektrode, an der sie vorbeigeführt wird, über ihren gesamten Umfangsbereich elektrisch zu isolieren, was jedoch auf nachteilige Weise einen erhöhten Herstellungsaufwand bedingt. Zudem können durch die Isolation für den Träger eines derartigen Brustgürtels unangenehme Erhebungen entstehen.

Eine Aufgabe der Erfindung besteht darin, einen Brustgürtel der eingangs genannten Art bereitzustellen, der unabhängig von der Anordnung und der Anzahl der vorgesehenen Elektroden unter jeglichen Einsatzbedingungen eine zuverlässige Signalübertragung zwischen Elektroden und Kontaktabschnitt bzw. Kontaktabschnitten sicherstellt. Insbesondere soll ein solcher Brustgürtel einfach und kostengünstig herzustellen und angenehm zu tragen sein.

Erfindungsgemäß wird diese Aufgabe durch die Merkmale des Patentanspruchs 1 und insbesondere dadurch gelöst,
- dass ein Gürtelabschnitt in Sandwichbauweise aufgebaut ist und zwei Isolierschichten aufweist, die zwischen zwei Textilschichten eingebettet sind,
- dass der Gürtelabschnitt zumindest zwei in Gürtellängsrichtung voneinander beabstandete Elektroden aufweist, wobei jeder Elektrode eine zwischen den beiden Isolierschichten eingebettete, zum jeweiligen Kontaktabschnitt führende elektrische Leitung zugeordnet ist, und
- dass die innere Isolierschicht sowie die innere Textilschicht miteinander fluchtende Durchbrechungen aufweisen, durch welche sich die Elektroden hindurch erstrecken.

Da weder die Leitungen noch die Isolierschichten aus textilem Material bestehen, können ihre Leitfähigkeits- bzw. Isoliereigenschaften durch das Eindringen von Schweiß nicht beeinträchtigt werden, so dass die vorstehend erwähnten, beim Stand der Technik gegebenen Nachteile vermieden werden. Durch das Einbetten der Isolierschichten mit den zwischen ihnen befindlichen Leitungen zwischen zwei Textilschichten wird jedoch dennoch der bei Brustgürteln gemäß dem Stand der Technik gegebene Tragekomfort erzielt. Die Haut einer den erfindungsgemäßen Brustgürtel benutzenden Person gelangt nämlich aufgrund der vorgesehenen Textilschichten mit Ausnahme der vergleichsweise kleinen Elektrodenbereiche ausschließlich mit textilem Material in Kontakt, was wesentlich zum Tragekomfort beiträgt.

Wenn zwischen Brustgürtel und Haut Schweiß entsteht, kann dieser lediglich in die innenseitige Textilschicht eindringen, die jedoch keinerlei Leitfähigkeits- oder Isoliereigenschaften aufweisen muss, da diese Leitfähigkeits- oder Isoliereigenschaften erfindungsgemäß ausschließlich von den innerhalb der Textilschichten befindlichen Leitungen bzw. Isolierschichten übernommen werden. Im Bereich der Elektroden hingegen ist die Entstehung von Schweiß sogar vorteilhaft, da dieser die Leitung von elektrischen Signalen zwischen Haut und Elektroden begünstigt.

Durch die erfindungsgemäße Sandwichbauweise lässt sich der Brustgürtel sehr einfach und kostengünstig herstellen, insbesondere wenn die einzelnen Schichten mittels eines thermischen Verfahrens miteinander verbunden werden. Wenn die Isolierschichten ungefähr gleich breit und die Textilschichten geringfügig breiter als die Isolierschichten ausgebildet werden, lässt sich ein Brustgürtel herstellen, der annähernd ebene Oberflächen aufweist, was innenseitig zu einer weiteren Verbesserung des Tragekomforts und außenseitig zu vorteilhaften Reinigungsmöglichkeiten führt. Wenn die Textilschichten geringfügig breiter sind als die Isolierschichten, können die Isolierschichten über den gesamten Umfang des Brustgürtels vom Material der Textilschichten bedeckt sein, so dass die gesamte Oberfläche des Brustgürtels mit Ausnahme der vergleichsweise kleinen Elektrodenbereiche von den Textilschichten gebildet wird.

Da sich die von den Elektroden zu dem Kontaktabschnitt führenden Leitungen zwischen zwei Isolierschichten befinden, sind die Leitungen zwangsläufig über ihren gesamten Umfang isoliert, so dass komplett ausgeschlossen werden kann, dass zwischen Leitungen und solchen Elektroden, an denen die Leitungen vorbeigeführt werden, Spannungsbrücken entstehen.

Es ist von Vorteil, wenn die außenseitige Isolierschicht sowie die außenseitige Textilschicht durchbrechungsfrei ausgebildet sind. Hierdurch ergibt sich eine weitestgehend glatte und ebene äußere Oberfläche des Brustgürtels, die besonders gut zu reinigen ist.

Der Tragekomfort des erfindungsgemäßen Brustgürtels wird zusätzlich verbessert, wenn die Bestandteile des erfindungsgemäß ausgebildeten Gürtelabschnitts elastisch ausgeführt sind. Ein derartiger Brustgürtel beengt seinen Träger nur minimal, da er Atem- und sonstige Bewegungen mit vollziehen kann.

Grundsätzlich ist es möglich, den Brustgürtel über seine gesamte Länge so auszubilden, wie den vorstehend erläuterten erfindungsgemäßen Gürtelabschnitt. Bevorzugt ist es jedoch, wenn nur ein oder zwei Abschnitte eines Brustgürtels erfindungsgemäß ausgebildet werden und zusätzlich ein weiterer elektroden- und leitungsfreier Gürtelabschnitt vorgesehen wird, der aus einem dehnbaren, insbesondere längenverstellbaren Textilmaterial besteht, dessen Elastizitätsmodul geringer ist als dasjenige des bzw. der anderen Gürtelabschnitte. Dieses geringere Elastizitätsmodul bedingt, dass der weitere Gürtelabschnitt besonders gut dehnbar ist, so dass ein solcher Gürtel letztlich einen noch höheren Tragekomfort bedingt. Die Endabschnitte eines solchen weiteren Gürtelabschnitts können zwischen den Textilschichten befestigt sein, wobei diese Befestigung bevorzugt mittels eines Heißklebeverfahrens bewerkstelligt wird.

Die Elektroden bestehen bevorzugt aus einem Elastomermaterial mit einem Ascheanteil, wobei der Ascheanteil bevorzugt mehr als 3% und insbesondere ungefähr 6% beträgt. Insbesondere bestehen die Elektroden aus einem Polymerisat aus Butadien und Acrylnitril (NBR). Der in den Elektroden enthaltene Ascheanteil kann als Hauptbestandteile folgende Stoffe aufweisen: Zn, Ca, S, Ni, Si, Fe, V und Mg.

Weiterhin können die Elektroden zusätzlich einen Silberchloridanteil aufweisen, was zu einer Verbesserung der Leiteigenschaften der Elektroden führt. Der Silberchloridanteil ist dabei bevorzugt kleiner als 10 Gewichtsprozent, wobei er auch deutlich kleiner als dieser Wert sein kann und dennoch den genannten Effekt der Verbesserung der Leiteigenschaften bewirkt.

Besonders vorteilhaft ist es, wenn die Leitungen zwischen den Elektroden und den Kontaktabschnitten aus dem gleichen Material bestehen wie die Elektroden. In diesem Fall lassen sich Elektroden und Leitungen in einem einzigen Arbeitsschritt besonders einfach herstellen, wobei die Leitungen hier bevorzugt die Form von schmalen Bändern besitzen, deren Dicke der Dicke der Elektroden entspricht. Bei dem erwähnten gemeinsamen Herstellungsprozess kann den Leitungen auch gleich die jeweils gewünschte geometrische Form verliehen werden, was insbesondere dann von Vorteil ist, wenn eine Leitung zwischen Elektrode und Kontaktabschnitt nicht geradlinig verläuft.

Alternativ ist es jedoch auch möglich, die Leitungen zwischen den Elektroden und den Kontaktabschnitten aus einem anderen Material zu fertigen als die Elektroden, wobei sich hier insbesondere die Verwendung flexibler Kupferleitungen anbietet.

Der Abstand zwischen der innenseitigen Elektrodenoberfläche und der Oberfläche der inneren Isolierschicht ist bevorzugt größer als die Dicke der inneren Textilschicht. Auf diese Weise wird erreicht, dass die Elektrode geringfügig, insbesondere etwas weniger als 1 mm, über die Oberfläche der inneren Isolierschicht herausragt, was beim Tragen des Brustgürtels dazu führt, dass eine solche Elektrode mit einer ausreichend großen Kraft vollflächig an die Haut des Trägers gedrückt wird, so dass eine gute Signalleitung gewährleistet ist.

Die Randbereiche der Textilschichten können mittels eines Klebeverfahrens miteinander verbunden werden. Weitere Bereiche der Textilschichten werden mittels eines Klebeverfahrens mit den Isolierschichten verbunden. Als Klebeverfahren eignen sich insbesondere Heißklebeverfahren.

Die innere Textilschicht kann quer zur Gürtellängsrichtung verlaufende Rillen aufweisen, die sich über die gesamte Gürtelbreite erstrecken. Wenn derartige Rillen im Bereich der Elektroden vorgesehen werden, wird hierdurch erreicht, dass oberhalb der Elektrode entstehender Schweiß auf vorteilhafte Weise in Richtung der Elektroden geführt wird, um dort die Leitfähigkeit zwischen Haut und Elektrode zu verbessern. Die unterhalb der Elektroden vorhandenen Rillen begünstigen ein Ableiten von entstehendem Schweiß nach unten.

Alternativ kann die innere Textilschicht auch parallel zur Gürtellängsrichtung verlaufende Rillen aufweisen. Derartige Längsrillen verhindern ein Verrutschen des Brustgürtels quer zu seiner Längserstreckung beim Tragen. Es ist auch denkbar, längs und quer verlaufende Rillen an der inneren Textilschicht miteinander zu kombinieren, wobei es vorteilhaft ist, die quer zur Gürtellängsrichtung verlaufenden Rillen im Bereich der Elektroden und die parallel zur Gürtellängsrichtung verlaufenden Rillen in allen anderen Bereichen des Gürtelabschnitts bzw. Brustgürtels vorzusehen. Die äußere Textilschicht ist auf ihrer Außenseite bevorzugt glatt und rillenfrei ausgebildet, um so wiederum eine gute Reinigungsmöglichkeit zu bieten.

Ein mit erfindungsgemäßen Gürtelabschnitten ausgebildeter Brustgürtel kann über seine gesamte Länge eine konstante Breite besitzen. Ebenso ist es jedoch möglich, denjenigen Bereich des Gürtelabschnitts, indem sich eine Elektrode und eine Leitung parallel zueinander erstrecken, breiter auszubilden als andere Gürtelabschnitte.

Die Isolierschichten, die Elektroden sowie die Leitungen des erfindungsgemäßen Gürtelabschnitts werden auf vorteilhafte Weise mittels eines Vulkanisiervorgangs miteinander verbunden.

Weitere bevorzugte Ausführungsformen der Erfindung können den Patentansprüchen, der Figurenbeschreibung und den Figuren entnommen werden.

Die Erfindung wird nachfolgend anhand eines Ausführungsbeispiels unter Bezugnahme auf die Figuren erläutert; in diesen zeigen:
- Fig. 1: eine Draufsicht auf zwei Gürtelabschnitte unter Weglassung der inneren Isolierschicht sowie der inneren Textilschicht, und
- Fig. 2: eine Draufsicht auf die Elektroden und Leitungselemente, welche in den Gürtelabschnitten gemäß Fig. 1 zum Einsatz gelangen.

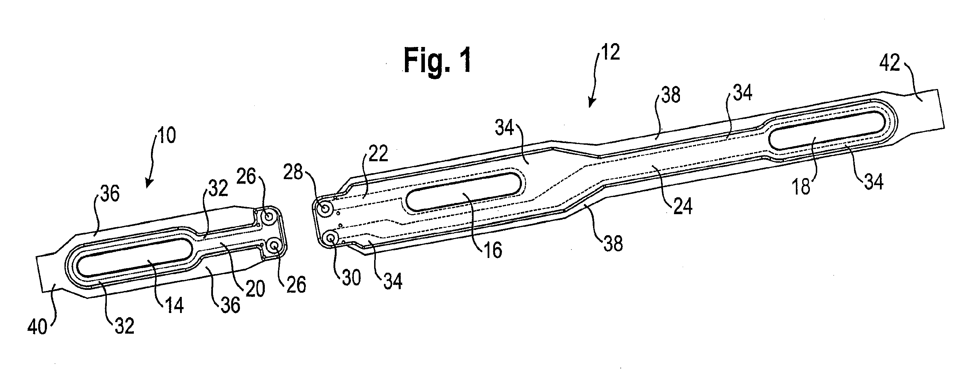

Fig. 1 zeigt zwei Gürtelabschnitte 10 und 12, wobei der Gürtelabschnitt 10 lediglich eine Elektrode 14, der Gürtelabschnitt 12 jedoch in erfindungsgemäßer Weise zwei Elektroden 16, 18 aufweist. Bei einem in Verwendung befindlichen Brustgürtel kommen die Elektroden 14, 16 im Brustbereich eines Patienten und die Elektrode 18 im Rückenbereich eines Patienten zu liegen.

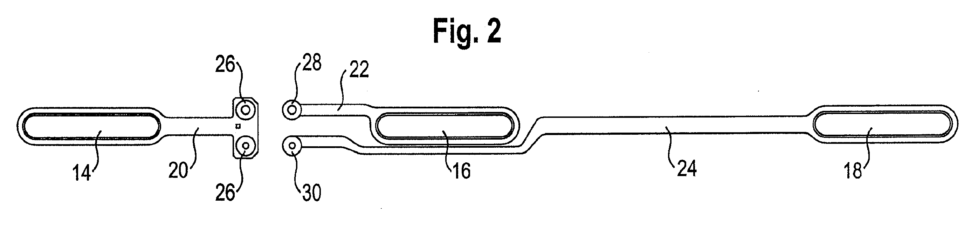

Die Elektroden 14, 16, 18 sind einstückig mit ihnen jeweils zugeordneten Leitungen 20, 22, 24 und diesen Leitungen 20, 22, 24 jeweils zugeordneten Kontaktabschnitten 26, 28, 30 ausgebildet. Die geometrische Anordnung der vorstehend erwähnten Bestandteile 14 bis 30 ist besonders gut aus Fig. 2 ersichtlich, welche zeigt, dass die der Elektrode 18 und dem Kontaktabschnitt 30 zugeordnete Leitung 24 zweifach abgewinkelt ausgebildet ist, damit sie an der Elektrode 16 mit Abstand und somit ohne elektrischen Kontakt vorbeigeführt werden kann.

Wie bereits erwähnt, sind die Elektroden 14, 16, 18, die Leitungen 20, 22, 24 sowie die Kontaktabschnitte 26, 28, 30 einstückig und somit aus ein und demselben Material gefertigt, so dass sich die drei in Fig. 2 dargestellten Einheiten, die jeweils aus einer Elektrode 14, 16, 18, einer Leitung 20, 22, 24 und einem Kontaktabschnitt 26, 28, 30 bestehen, jeweils in einem einzigen Herstellungsschritt fertigen lassen.

Die Elektrode 14 mit der ihr zugeordneten Leitung 20 und dem ihr zugeordneten Kontaktabschnitt 26 ist mit einer äußeren Isolierschicht 32 klebend verbunden, wobei der Umriss der äußeren Isolierschicht 32 etwas größer bemessen ist als der Umriss der einstückig miteinander ausgebildeten Elemente 14, 20, 26. Auf die Elemente 14, 20, 26 und die äußere Isolierschicht 32 wird im weiteren Herstellungsverfahren eine in Fig. 1 nicht dargestellte innere Isolierschicht aufgeklebt, deren Umriss identisch zum Umriss der äußeren Isolierschicht 32 ausgebildet ist. Durch das Verkleben der Isolierschichten, insbesondere in ihrem äußeren Umfangsbereich, wird erreicht, dass die elektrisch leitenden Elemente 14, 20, 26 von der äußeren Isolierschicht 32 und der nicht dargestellten inneren Isolierschicht komplett umschlossen und somit vollständig elektrisch isoliert sind. Einzige Ausnahme ist hierbei der Elektrodenbereich 14 sowie der Kontaktabschnitt 26. In diesen Bereichen weist die nicht dargestellte innere Isolierschicht Aussparungen auf, deren Abmessungen weitgehend identisch zu den Abmessungen der Elektrode 14 und des Kontaktabschnitts 26 sind, so dass die Elektrode 14 in Kontakt mit der Haut eines Patienten gelangen kann und der Kontaktabschnitt 26 elektrisch leitend mit einer nicht dargestellten Sendeeinheit gekoppelt werden kann.

In entsprechender Weise sind die Elemente 16 bis 30 auf eine gemeinsame äußere Isolierschicht 34 im Bereich des zweiten Gürtelabschnitts 12 aufgeklebt, wobei der äußere Umriss dieser äußeren Isolierschicht 34 größer bemessen ist als die äußere Umfangslinie der Gesamtheit der Elemente 16 bis 30. Auf die äußere Isolierschicht 34 wird wiederum eine nicht dargestellte, gemeinsame innere Isolierschicht geklebt, deren Abmessungen mit den Abmessungen der äußeren Isolierschicht 34 übereinstimmen, so dass die Gesamtheit aus äußerer Isolierschicht 34 und aufgeklebter innerer Isolierschicht die Elemente 16 bis 30 vollständig elektrisch isoliert. Einzige Ausnahme sind wiederum die Elektrodenbereiche 16, 18 sowie die Kontaktabschnitte 28, 30 in denen die nicht dargestellte innere Isolierschicht größenmäßig angepasste Aussparungen aufweist. Die äußere Isolierschicht 34 gemeinsam mit der mit ihr verklebten inneren Isolierschicht stellt hier zugleich sicher, dass kein elektrischer Kontakt zwischen der Elektrode 16 und der Leitung 24 existieren kann.

Die äußeren Isolierschichten 32, 34 werden mit ihrer den Elementen 14 bis 30 abgewandten Seite mit jeweils einer äußeren Textilschicht 36, 38 verklebt. Die äußeren Umfangslinien der äußeren Textilschichten 36, 38 sind etwas größer bemessen, als die äußeren Umfangslinien der äußeren Isolierschichten 32, 34. Lediglich in den Bereichen der Kontaktabschnitte 26, 28, 30 ist keine äußere Textilschicht 36, 38 vorhanden. Auf die äußeren Textilschichten 36, 38 werden nicht dargestellte innere Textilschichten geklebt, die die gleichen Abmessungen aufweisen wie die äußeren Textilschichten 36, 38. Die inneren Textilschichten besitzen jedoch im Unterschied zu den äußeren Textilschichten 36, 38 im Bereich der Elektroden 14, 16, 18 Aussparungen, so dass die Elektroden 14, 16, 18 mit der Haut eines Patienten in Kontakt treten können. Im Bereich der Kontaktabschnitte 26, 28, 30 sind keine Aussparungen der Textilschichten nötig, da in diesen Bereichen keine Textilschichten vorhanden sind. Somit umschließen jeweils eine äußere und eine innere Textilschicht eine Einheit aus innerer und äußerer Isolierschicht vollständig mit Ausnahme der jeweiligen Kontaktabschnitte26, 28, 30.

Die inneren und äußeren Textilschichten 36, 38 besitzen an ihren den Kontaktabschnitten 26 bzw. 28, 30 abgewandten Enden Befestigungslaschen 40, 42. Jede dieser Befestigungslaschen 40, 42 wird mit jeweils einem Ende eines nicht dargestellten gut dehnbaren, längenverstellbaren Textilbands verbunden, so dass letztlich ein kompletter Gürtel erhalten wird, der über eine nicht dargestellte Sendeeinheit geschlossen werden kann, indem die Sendeeinheit mit den Kontaktabschnitten 26 bzw. 28, 30 mechanisch und elektrisch gekoppelt wird.

Die Elektroden 14, 16, 18 stehen in einer Richtung senkrecht zu den Isolierschichten 32, 34 über die Oberfläche der Leitungen 20, 22, 24 in einem solchen Maße hervor, dass sie sich komplett durch die nicht dargestellte innere Isolierschicht sowie die nicht dargestellte innere Textilschicht hindurch erstrecken und sogar etwas über die Oberfläche der inneren Textilschicht herausragen. Auf diese Weise wird sichergestellt, dass die Elektroden 14, 16, 18 bei angelegtem Brustgürtel einen guten, vollflächigen, elektrischen Kontakt zur Haut des jeweiligen Patienten besitzen.

### Bezugszeichenliste

- 10, 12: Gürtelabschnitt
- 14, 16, 18: Elektrode
- 20, 22, 24: Leitung
- 26, 28, 30: Kontaktabschnitt
- 32, 34: äußere Isolierschicht
- 36, 38: äußere Textilschicht
- 40, 42: Befestigungslasche

## Patentansprüche

1. Brustgürtel zur Aufnahme von EKG-Signalen mit einer Innen- und einer Außenseite sowie zumindest zwei innenseitig angeordneten, elektrisch leitenden, flexiblen Elektroden (16, 18), welche ein Polymer enthalten und über Leitungen (22, 24) mit je einem Kontaktabschnitt (28, 30) verbunden sind,
**dadurch gekennzeichnet ,**
**dass** ein Gürtelabschnitt (12) in Sandwichbauweise aufgebaut ist und zwei Isolierschichten (34) aufweist, die zwischen zwei Textilschichten (38) eingebettet sind,
**dass** der Gürtelabschnitt (12) zwei in Gürtellängsrichtung voneinander beabstandete Elektroden (16, 18) aufweist, wobei jeder Elektrode (16, 18) eine zwischen den beiden Isolierschichten (34) eingebettete, zum jeweiligen Kontaktabschnitt (28, 30) führende elektrische Leitung (22, 24) zugeordnet ist, und
**dass** die innere Isolierschicht sowie die innenseitige Textilschicht miteinander fluchtende Durchbrechungen aufweisen, durch welche sich die Elektroden (16, 18) hindurch erstrecken.

2. Brustgürtel nach Anspruch 1,
**dadurch gekennzeichnet ,**
**dass** die außenseitige Isolierschicht (34) sowie die außenseitige Textilschicht (38) durchbrechungsfrei ausgebildet sind.

3. Brustgürtel nach einem der vorhergehenden Ansprüche,
**dadurch gekennzeichnet ,**
**dass** alle Bestandteile des Gürtelabschnitts (12) elastisch ausgebildet sind.

4. Brustgürtel nach einem der vorhergehenden Ansprüche,
**dadurch gekennzeichnet ,**
**dass** die Elektroden (16, 18) aus einem Elastomermaterial mit einem Ascheanteil bestehen, wobei der Ascheanteil bevorzugt mehr als 3% und insbesondere ungefähr 6% beträgt, und/oder dass die Elektroden (16, 18) aus einem Polymerisat aus Butadien und Acrylnitril (NBR) bestehen.

5. Brustgürtel nach Anspruch 4,
**dadurch gekennzeichnet ,**
**dass** der in den Elektroden (16, 18) enthaltene Ascheanteil als Hauptbestandteile folgende Stoffe aufweist: Zn, Ca, S, Ni, Si, Fe, V und Mg.

6. Brustgürtel nach einem der Ansprüche 4 oder 5,
**dadurch gekennzeichnet ,**
**dass** die Elektroden (16, 18) zusätzlich einen Silberchloridanteil aufweisen.

7. Brustgürtel nach einem der vorhergehenden Ansprüche,
**dadurch gekennzeichnet ,**
**dass** die Leitungen (22, 24) aus dem gleichen Material bestehen wie die Elektroden (16, 18).

8. Brustgürtel nach einem der vorhergehenden Ansprüche,
**dadurch gekennzeichnet ,**
**dass** der Abstand zwischen der innenseitigen Elektrodenoberfläche und der Oberfläche der inneren Isolierschicht größer ist als die Dicke der inneren Textilschicht.

9. Brustgürtel nach einem der vorhergehenden Ansprüche,
**dadurch gekennzeichnet ,**
**dass** die Randbereiche der Textilschichten (38) mittels eines Klebeverfahrens miteinander verbunden sind und weitere Bereiche der Textilschichten (38) mittels eines Klebeverfahrens mit den Isolierschichten verbunden sind.

10. Brustgürtel nach einem der vorhergehenden Ansprüche,
**dadurch gekennzeichnet ,**
**dass** die innere Textilschicht quer zur Gürtellängsrichtung verlaufende Rillen aufweist, die sich über die gesamte Gürtelbreite erstrecken, und/oder
**dass** die innere Textilschicht parallel zur Gürtellängsrichtung verlaufende Rillen aufweist.

11. Brustgürtel nach einem der vorhergehenden Ansprüche,
**dadurch gekennzeichnet ,**
**dass** die Isolierschichten (34), die Elektroden (16, 18) sowie die Leitungen (22, 24) des Gürtelabschnitts (12) mittels eines Vulkanisiervorgangs miteinander verbunden sind.

12. Brustgürtel nach einem der vorhergehenden Ansprüche,
**dadurch gekennzeichnet ,**
**dass** ein weiterer, elektroden- und leitungsfreier Gürtelabschnitt aus einem dehnbaren, längenverstellbaren Textilmaterial besteht, dessen Elastizitätsmodul geringer ist als dasjenige anderer Gürtelabschnitte (10, 12).
